# EUROPEAN PATENT APPLICATION

(11) **EP 3 834 888 A2**
(43) Date of publication of application: **16.06.2021**
(21) Application number: 19847628.5
(22) Date of filing: 12.08.2019
(51) Int. Cl.: A61P 25/32, A61K 31/05, A61K 31/41

(54) **AGENTS FOR TREATMENT OF ALCOHOL USE DISORDER**

(30) Priority: 10.08.2018 ES 201830824; 10.08.2018 ES 201830825
(71) Applicant: Servicio Andaluz de Salud, 41071 Sevilla (ES)
(72) Inventor: RODRÍGUEZ DE FONSECA, Fernando, 41071 Sevilla (ES); DECARA DEL OLMO, Juan, 41071 Sevilla (ES); SERRANO CRIADO, Antonia, 41071 Sevilla (ES); ALEN FARIÑAS, Francisco, 41071 Sevilla (ES)
(74) Representative: Hoffmann Eitle
(86) International application number: PCT/ES2019/070565
(87) International publication number: WO 2020/030843

(57) **Abstract**

The present invention relates to the use of PPARα agonist compounds for preventing, alleviating, improving and/or treating alcohol use disorder.

## Description

### TECHNICAL FIELD OF THE INVENTION

The present invention is comprised in the field of medicine and pharmacy and relates to the use of PPARα (*peroxisome proliferator-activated receptor alpha*) agonist compounds for preventing, alleviating, improving, and/or treating alcohol use disorder, in combination with at least one CB1 receptor (cannabinoid receptor) antagonist and/or with a PPAPγ (peroxisome proliferator-activated receptor gamma) agonist.

### BACKGROUND OF THE INVENTION

Alcohol consumption is widespread in society. According to the latest Survey regarding Alcohol and other Drugs in Spain (*Encuesta Sobre Alcohol y Otras Drogas en España*) (National Drug Plan (*Plan Nacional Sobre Drogas*), EDADES 2015-2016), 77% of the population between 15 and 64 years of age (82.9% of men and 72.1% of women) have consumed alcohol in the last year, and alcohol is one of the drugs whose consumption begins at an earlier age (16.6 years old) together with tobacco (*Plan Nacional Sobre Drogas,* EDADES 2015-2016). In this sense, more than 75% of young Spanish secondary school students between 14 and 18 years of age consume alcohol, -32% presents a binge drinking or consumption risk and -22% has been inebriated in the last month (*Plan Nacional Sobre Drogas,* ESTUDES 2014/2015). Moreover, based on the epidemiological study ESEMeD-España (Haro et al. 2006. Med Clin (Barc.), 126(12):445-51), alcoholism or alcohol use disorder (AUD) is one of the most prevalent lifelong disorders in Spain, estimated at 3.6%, and it is more common in men (6.47%) than in women (0.96%). It should be noted that a period is used to separate the integer from the decimal.

It is one of the main causes of morbidity and mortality and few medicinal products have been made available on the market to help combat this devastating chronic disorder. Multiple signalling systems have been linked to alcohol use, including dopamine, serotonin, noradrenaline, glutamate, opioid peptides, nociceptin, or corticotropin-releasing factor [Koob GF. 2014. Handb Clin Neurol. 125:33-54]. Chronic alcohol abuse results in the imbalance of these neurotransmitter systems, and the therapies available today have tried to correct them. In fact, this is the case of opiate antagonists naltrexone and nalmefene, or the NMDA glutamate receptor antagonist acamprosate. However, the medicinal products currently approved only target patient subpopulations who respond to these drugs and they encompass only one part of all the patients affected by alcohol use disorder. Alternative therapies for covering the therapeutic needs of patients with AUD are lacking.

Among the multiple signalling systems affected by alcohol, lipids related to endocannabinoids (anandamide, 2-AG, acylethanolamides, and other arachidonic acid derivatives) have been proven to be involved in different steps of the alcohol addiction cycle, including intoxication, alcohol-seeking behavior, and consumption relapse after a period of abstinence. They are also involved in anxiety and emotional disorders associated with alcohol addiction, alcohol-induced neuroinflammation, or alcohol-related liver disease (Serrano et al. Neuropsychopharmacology. 43, pages 1840-1850 (2018)). Moreover, genetic studies have identified genetic variants in the genes encoding the proteins involved in endocannabinoid signalling that were associated with alcohol use disorder. This finding confirms the important role of this signalling system in the initiation and maintenance of alcohol use.

Based on these discoveries, multiple medicinal chemistry programs have produced specific drugs related to the endocannabinoid system which have been used in preclinical models of alcohol use disorder. They include:
a) cannabinoid CB1 receptor agonists and antagonists,
b) cannabinoid CB2 receptor agonists and antagonists,
c) peroxisome proliferator-activated receptor alpha (PPARα) agonists,
d) peroxisome proliferator-activated receptor gamma (PPARy) agonists,
e) fatty acid amide hydrolase (FAAH, one of the main endocannabinoid-degrading enzymes) inhibitors, or
f) endocannabinoid reuptake inhibitors (such as AM404).

However, the strategy of using a combination treatment has not been tested to date, probably due to the fact that the possible undesired side effects derived from the use of two independent chemical entities could be greater than the possible undesired side effects derived from using a single drug.

### DESCRIPTION OF THE FIGURES

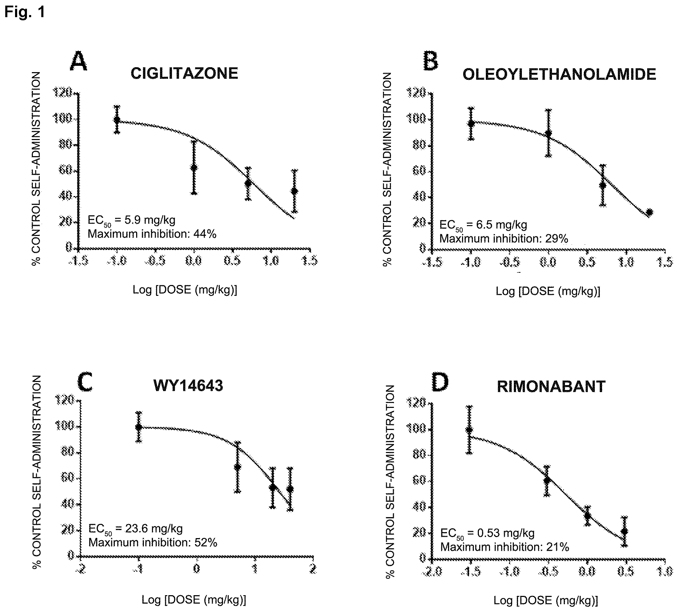

**Figure 1****.** Dose-dependent inhibition of the alcohol self-administration in rats after the acute administration of OLHHA. The results are presented as percentages of the self-administration responses of the control group versus Log [OLHHA dose, in mg/kg]. EC50 values were calculated by means of non-linear regression. **A.** Effect of PPAPγ agonist ciglitazone; **B.** Endogenous PPARα ligand oleoylethanolamide; C. Synthetic PPAPγ agonist WY14643; D. cannabinoid CB1 receptor antagonist/inverse agonist rimonabant.

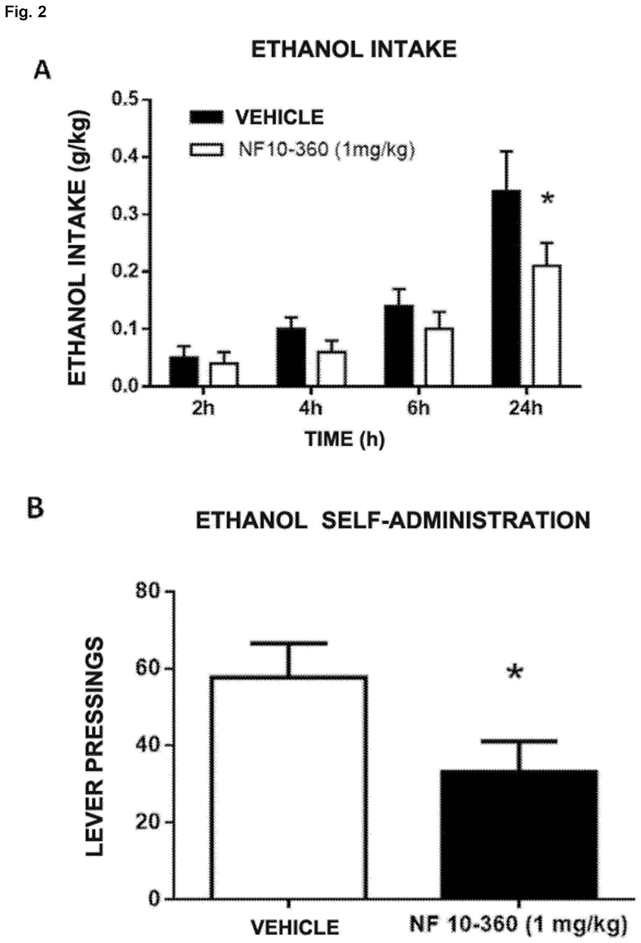

**Figure 2****. A.** Effects of 1 mg/kg of NF 10-360 on the use of the two-bottle choice paradigm in Wistar rats. **B.** Effect of 1 mg/kg of NF 10-360 on alcohol self-administration in Wistar rats. The data represents means ± SEM. * P <0.05 vs vehicle.

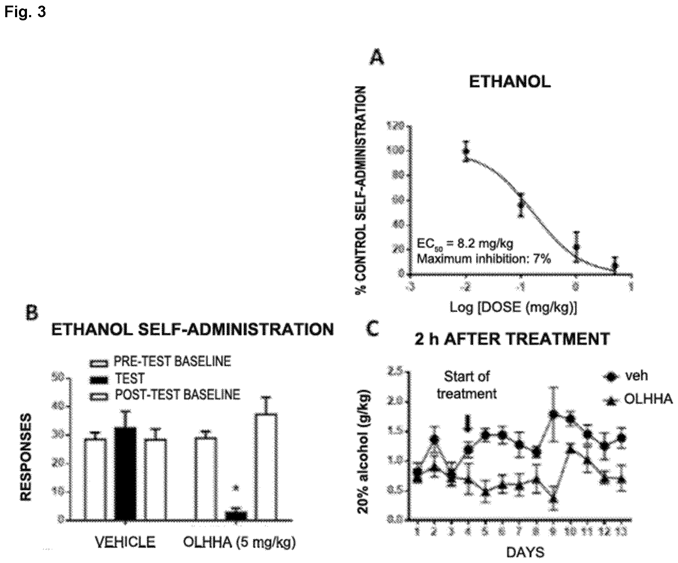

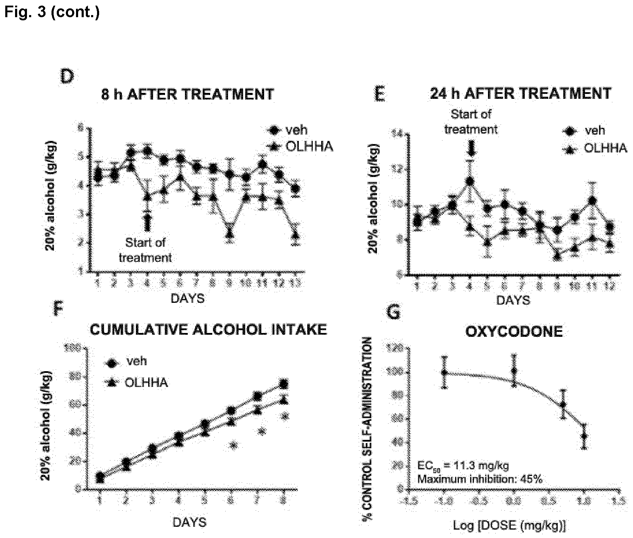

**Figure 3****. A.** Figures 3A and 3B show the effects of the PPARα agonist and CB1 receptor antagonist OLHHA on ethanol self-administration (Figures 3A and 3B) in adult male Wistar rats. Figures 3C to 3F show the effects of the daily administration of 5 mg/kg of OLHHA 2, 8, and 24 hours after administration. OLHHA reduced alcohol consumption 2 hours after the injection, F (1.14) = 39.5, P <0.0001 (Figure 3C). This effect was also observed 8 hours after the administration of OLHHA, F (1.14) = 23.9, P <0.001 (Figure 3D), and almost disappeared after 24 h, F (1.14) = 4.7, P = 0.047 (Figure 3E). Total alcohol intake throughout treatment with OLHHA was significantly reduced, in any case, F (1.14) = 5.1, P <0.05 (Figure 3F). Lastly, Figure 3G shows the effects of OLHHA on self-administration of the opiate oxycodone in Long-Evans rats. EC50 values were measured using non-linear regression. The data represents means ± SEM. * P <0.05 vs vehicle.

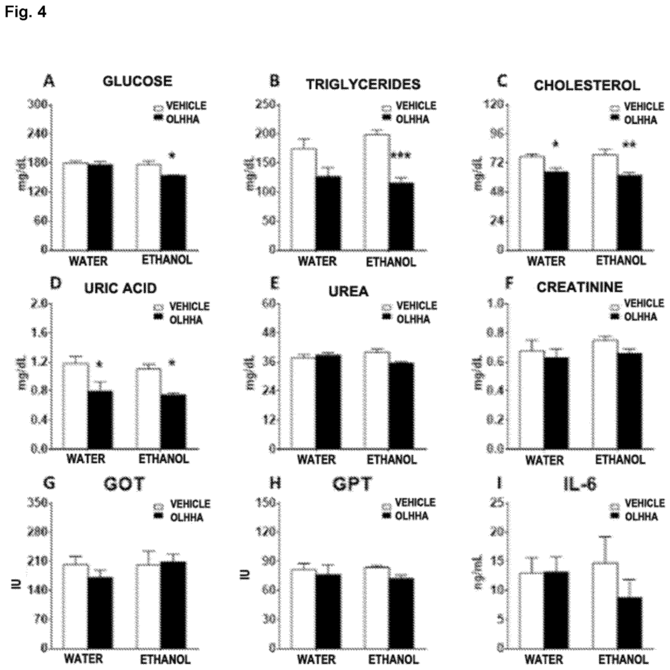

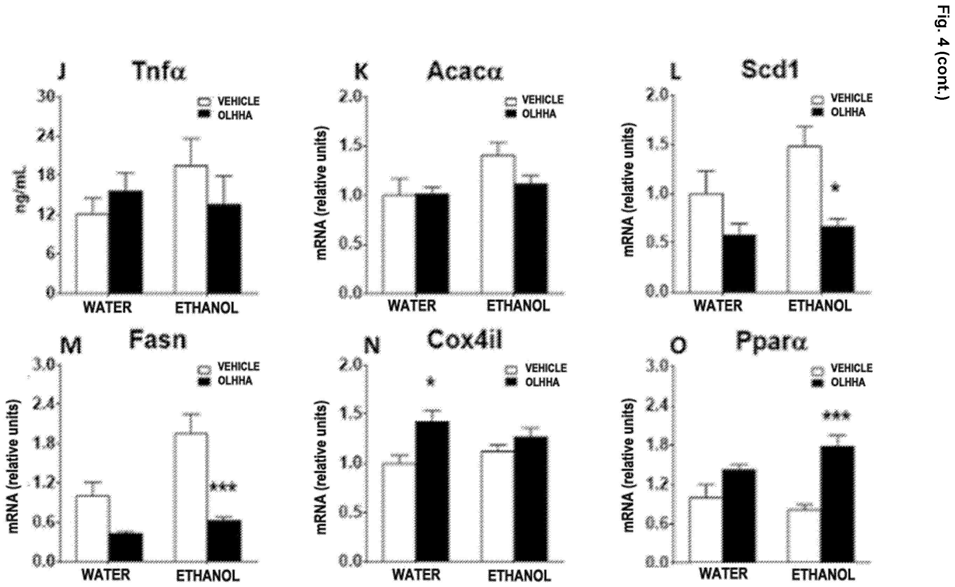

**Figure 4****.** Plasma levels of glucose (A), triglycerides (B), cholesterol (C), uric acid (D), urea (E), and creatinine (F) in msP rats or 20% water, and treated daily with a vehicle or OLHHA 5 mg/kg. Transaminase activity (panels G and H) and plasma concentrations of cytokines IL-6 (I) and TNFα (J) in the same animals. Panels K to O, mRNA expression in the liver of the main lipogenic enzymes [acetyl coenzyme A carboxylase (ACACa, panel K), stearoyl-coenzyme A desaturase 1 (SCD-1, panel L), and fatty acid synthase (Fasn, panel M)], cytochrome C oxidase 4 (N), and the nuclear receptor PPARα (O) after treatment with OLHHA. The data represents means ± SEM. * P <0.05, ** P <0.01, *** P <0.001 vs respective control.

### DESCRIPTION OF THE INVENTION

The authors of the present invention have demonstrated, as shown in the examples, that drugs combining two pharmacological profiles have additive/synergistic pharmacological effects, reducing alcohol intake.

### MEDICAL USES OF THE COMPOUNDS OF THE INVENTION

Therefore, a **first aspect** of the invention relates to a combined preparation comprising at least one PPARα agonist and at least one CB1 receptor antagonist, hereinafter first combined preparation of the invention, for use in preventing, alleviating, improving, and/or treating alcohol use disorder.

In the present invention, alcohol use disorder includes both the effects of use and the effects of abusive consumption.

The term "combined preparation," or also referred to as "juxtaposition," herein means that the components of the combined preparation do not have to be present as a blend, for example in a true composition, to be available for their combined, separate, or time-sequential application. Therefore, the expression "juxtaposed" implies that it is not necessarily a true combination in view of the physical separation of the components.

In a preferred embodiment of this aspect of the invention, the first combined preparation of the invention comprises at least one PPARα agonist and at least one CB1 receptor antagonist, preferably as the only active ingredients, although said preparation may additionally comprise pharmacologically acceptable excipients and vehicles.

In another preferred embodiment of this aspect of the invention, the PPARα agonist of the first combined preparation of the invention is selected from the list consisting of: clofibrate, gemfibrozil, fenofibrate, elafibranor, prasterone, ciprofibrate, oleoylethanolamide, CP 775146, GW 7647, WY 14643, or any salts, preferably any pharmaceutically acceptable salt, or pharmaceutically acceptable esters, tautomers, polymorphs, or hydrates, or an isomer, prodrugs, solvates, or analogs thereof, or any combinations thereof.

In another preferred embodiment of this aspect of the invention, the CB1 receptor antagonist of the first combined preparation of the invention is selected from antagonists AVE-1625, CP-945598, SLV319, V24343 AM 251, AM 4113, AM 6545, CP 945598 hydrochloride, MJ 15, NIDA 41020, PF 514273, (±)-SLV 319, propacetamol, tetrahydrocannabivarin, or inverse agonists selected from AM 281, hemopressin, LY 320135, SR 141716A, TC-C 14G, or any salts, preferably any pharmaceutically acceptable salt, pharmaceutically acceptable esters, tautomers, polymorphs, hydrates, or an isomer, prodrugs, derivatives, solvates, or analogs thereof, or any combinations thereof.

Although the combinatorial use of two drugs could provide additive/synergistic actions, the possible undesired side effects derived from the use of two independent chemical entities could be greater than the possible undesired side effects derived from the use of a single drug with a double profile. The identification and use of dual ligands, i.e., individual molecules acting on two different pharmacological targets, may provide advantages in terms of safety and efficacy.

Therefore, a more preferred embodiment of the first aspect of the invention relates to a compound simultaneously exhibiting PPARα agonist activity and CB1 receptor antagonist activity, hereinafter first compound of the invention, for use in preventing, alleviating, improving, and/or treating alcohol use disorder. More preferably, the compound is the compound of formula (I) or any salts, preferably any pharmaceutically acceptable salt, or pharmaceutically acceptable esters, tautomers, polymorphs, or hydrates, or isomers, prodrugs, solvates, or analogs thereof, or any combinations thereof:

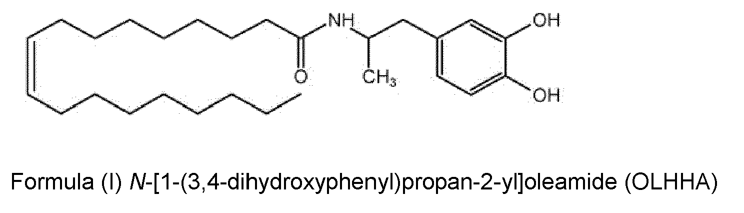

### N-[1-(3,4-dihydroxyphenyl)propan-2-yl]oleamide (OLHHA)

Other compounds simultaneously exhibiting PPARα agonist activity and CB1 receptor antagonist activity are included in formula (III) 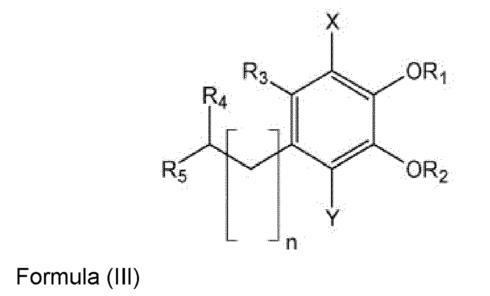 wherein
X and Y can be independently identical or different and are selected from H, halogen, and methyl;
n is an integer from 1 to 4;
R₁ and R₂ can be independently identical or different and are selected from H and a C₁-C₆ alkyl, or they can be attached by a single bond between the two oxygen atoms, forming a new cycle;
R₃ is selected from H, C₁-C₆ alkyl, and C₁-C₄ alkenyl;
R₄ is selected from H, halogen, and C₁-C₄ alkyl;
R₅ is a compound of general formula (IV): 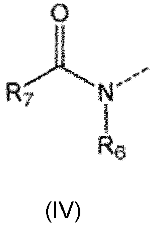 wherein:
R₆ is selected from H and C₁-C₄ alkyl;
R₇ is selected from C₈-C₃₀ alkyl and C₈-C₃₀ alkenyl;
or any salts, preferably any pharmaceutically acceptable salt, or pharmaceutically acceptable esters, tautomers, polymorphs, or hydrates, or an isomer, prodrugs, solvates, or analogs thereof, or any combinations thereof.

Furthermore and independently of what has previously been described, the authors of the present invention demonstrate that simultaneous activation of the PPARα and PPAPγ receptors gives rise to an additive/synergistic effect on reducing alcohol-seeking behavior.

Therefore, a **second aspect** of the invention relates to a combined preparation comprising at least one PPARα agonist and at least one PPAPγ agonist, hereinafter second combined preparation of the invention, for use in preventing, alleviating, improving, and/or treating alcohol use disorder.

In a preferred embodiment of this aspect of the invention, the second combined preparation of the invention comprises at least one PPARα agonist and at least one PPAPγ agonist, preferably as the only active ingredients, although said preparation may additionally comprise pharmacologically acceptable excipients and vehicles.

In another preferred embodiment of this aspect of the invention, the PPARα agonist of the second combined preparation of the invention is selected from the list consisting of: clofibrate, gemfibrozil, fenofibrate, elafibranor, prasterone, ciprofibrate, oleoylethanolamide, CP 775146, GW 7647, WY 14643, or any salts, preferably any pharmaceutically acceptable salt, or pharmaceutically acceptable esters, tautomers, polymorphs, or hydrates, or isomers, prodrugs, solvates, or analogs thereof, or any combinations thereof.

In another preferred embodiment of this aspect of the invention, the PPAPγ agonist of the second combined preparation of the invention is selected from the list consisting of: (2S)-2-(4-chlorophenoxy)-3-phenylpropanoic acid, (2S)-2-(biphenyl-4-yloxy)-3-phenylpropanoic acid, (2S)-2-ethoxy-3-{4-[2-(10H-phenoxazin-10-yl)ethoxy]phenyl}propanoic acid, (S)-3-(4-(2-carbazol-9-yl-ethoxy)-phenyl)-2-ethoxy-propionic acid, 2-chloro-5-nitro-N-phenylbenzamide, 2-{5-[3-(7-propyl-3-trifluoromethylbenzo[D]isoxazol-6-yloxy)propoxy]indol-1-yl}ethanoic acid, 3-(5-methoxy-1H-indol-3-yl)propanoic acid, 3-[5-(2-nitropent-1-en-1-yl)furan-2-yl]benzoic acid, 3-fluoro-N-[1-(4-fluorophenyl)-3-(2-thienyl)-1H-pyrazol-5-yl]benzenesulfonamide, balsalazide, glipizide, mesalazine, mitiglinide, nateglinide, repaglinide, sulfasalazine, T131, telmisartan, baroxolone, thiazolidinone, 15-deoxy-delta 12,14-prostaglandin J2, S26948, nTZDpa, LG 100754, GW 1929 hydrochloride, edaglitazone, ciglitazone, inolitazone, SR 2595, GW1929, or any salts, preferably any pharmaceutically acceptable salt, or pharmaceutically acceptable esters, tautomers, polymorphs, or hydrates, or isomers, prodrugs, solvates, or analogs thereof, or any combinations thereof.

Although the combinatorial use of two drugs could provide additive/synergistic actions, the possible undesired side effects derived from the use of two independent chemical entities could be greater than the possible undesired side effects derived from the use of a single drug with a double profile. The identification and use of dual ligands, i.e., individual molecules acting on two different pharmacological targets, may provide advantages in terms of safety and efficacy.

Therefore, a more preferred embodiment of the second aspect of the invention relates to a compound simultaneously exhibiting PPARα agonist activity and PPAPγ agonist activity, hereinafter second compound of the invention, for use in preventing, alleviating, improving, and/or treating alcohol use disorder. More preferably, the second compound of the invention is selected from the list consisting of indeglitazar, sodelglitazar, aleglitazar, fenofibrate, elafibranor, indomethacin, reglixane, bezafibrate, ibuprofen, icosapent, pioglitazone, rosiglitazone, troglitazone, muraglitazar, BMS 687453, WY-14643 (pirinixic acid), LT175, or any salts, preferably any pharmaceutically acceptable salt, or pharmaceutically acceptable esters, tautomers, polymorphs, or hydrates, or isomers, prodrugs, solvates, or analogs thereof, or any combinations thereof.

Even more preferably, the second compound of the invention is the compound of formula (II) or any salts, preferably any pharmaceutically acceptable salt, or pharmaceutically acceptable esters, tautomers, polymorphs, or hydrates, or isomers, prodrugs, solvates, or analogs thereof, or any combinations thereof.

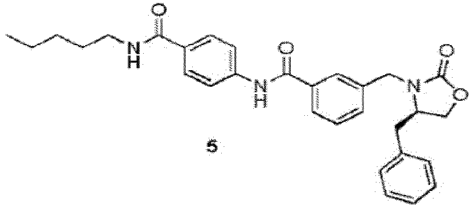

The name of the compound of formula (II) is 3-((4-benzyl-2-oxooxazolidin-3-yl)methyl)-N-(4-(pentylcarbamoyl)phenyl)benzamide (NF 10-360)

Other compounds simultaneously exhibiting PPARα agonist activity and PPAPγ agonist activity are included in formula (V): 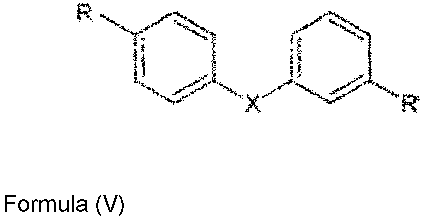 wherein:
- X is a secondary amide group which can be in two different positions, X1 and X2: 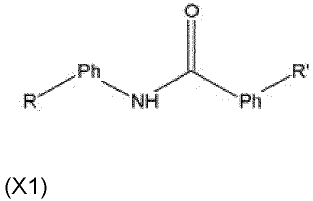 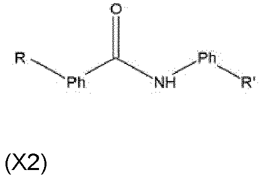
- R is a structure selected from 1 to 9 (R₁, R₂, R₃, R₄, R₅, R₆, R₇, R₈, and R₉):
It should be noted that the two signs (Ph) indicated above for X1 and X2 refer to the aromatic rings identified in formula (V) and separated by group X.

| | |
|---|---|
| R | |
| 1. | 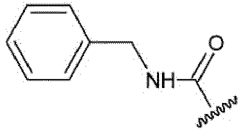 |
| 2. | 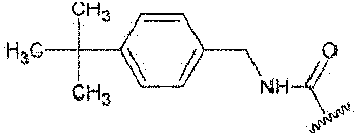 |
| 3. | 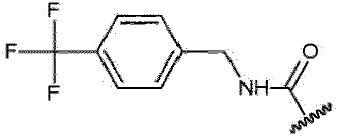 |
| 4. | 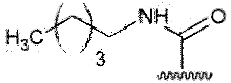 |
| 5. | 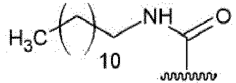 |
| 6. | 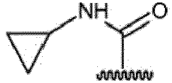 |
| 7. | 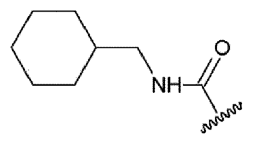 |
| 8. | 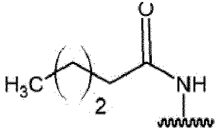 |
| 9. | 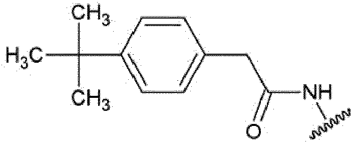 |

- R' is a structure selected from 1 to 4 (R'₁, R'₂, R'₃, and R'₄):

| R' | |
|---|---|
| 1. | 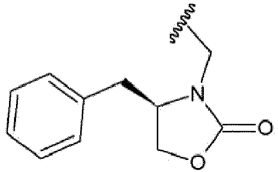 |
| 2. | 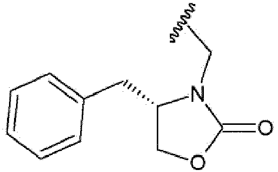 |
| 3. | 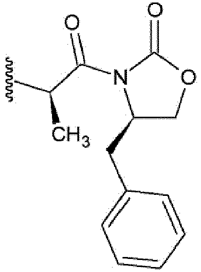 |
| 4. | 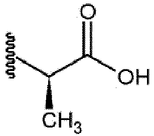 |

In a more preferred embodiment of this aspect, the compounds will be formed by combination R-Ph-X-Ph-R', wherein:
- For X₁ and R'₁, R will preferably be: R₁, R₂, R₃, R₄, R₅, R₆, and R₇
- For X₁ and R'₂, R will preferably be: R₂, R₃, and R₄
- For X₂ and R'₃, R will preferably be: R₈ and R₉
- For X₂ and R'₄, R will preferably be: R₈ and R₉

As indicated, any of the compounds of the present invention can include isomers, depending on the presence of multiple bonds, including optical isomers or enantiomers, depending on the presence of chiral centers. The individual isomers, enantiomers or diastereoisomers and the mixtures thereof fall within the scope of the present invention, i.e., the term isomer also refers to any mixture of isomers, such as diastereomers, racemic isomers, etc., including their optically active isomers or the mixtures thereof in different proportions. The individual enantiomers or diastereoisomers, as well as the mixtures thereof, can be separated by means of conventional techniques.

The prodrugs of any of the compounds of the invention also fall within the scope of this invention. As it is used herein, the term "prodrug" comprises any derivative of a compound of the invention, such as, for example, derivatives of those compounds of formula (I), where nonlimiting examples include: esters (including carboxylic acid esters, amino acid esters, phosphate esters, sulfonate esters of metal salts, etc.), carbamates, amides, etc., which when administered to an individual can be directly or indirectly transformed into said compound of the invention in the mentioned individual. Advantageously, said derivative is a compound which increases the bioavailability of the compound of the invention when it is administered to an individual or potentiates the release of the compound of the invention in a biological compartment. The nature of said derivative is not critical provided that it can be administered to an individual and provide the compound of the invention in a biological compartment of an individual. The preparation of said prodrug can be carried out by means of conventional methods known to those skilled in the art.

As it is used herein, the term "analog" includes both pharmaceutically acceptable compounds, i.e., analogs of the compound of the invention that can be used in the preparation of a medicinal product or food compositions, and non-non-pharmaceutically acceptable derivatives, since these derivatives can be useful in the preparation of pharmaceutically acceptable derivatives.

The compounds of the invention can be in crystalline form as free compounds or as solvates. In this sense, as it is used herein, the term "solvate" includes both pharmaceutically acceptable solvates, i.e., solvates of the compound of the invention that can be used in the preparation of a medicinal product, and non-pharmaceutically acceptable solvates, which can be useful in the preparation of pharmaceutically acceptable solvates or salts. The nature of the pharmaceutically acceptable solvate is not critical provided that it is pharmaceutically acceptable. In a particular embodiment, the solvate is a hydrate. The solvates can be obtained by conventional solvation methods known to those skilled in the art.

For their application in therapy, the compounds of the invention, the salts, prodrugs, or solvates thereof, will preferably be in a pharmaceutically acceptable or substantially pure form, i.e., they will have a pharmaceutically acceptable level of purity, excluding the usual pharmaceutical additives such as diluents and carriers, and not including material considered toxic at normal dosage levels. The levels of purity for the active ingredient are preferably greater than 50%, more preferably greater than 70%, and still more preferably greater than 90%. In a preferred embodiment, they are greater than 95% of the compound of the invention or the salts, solvates, or prodrugs thereof.

### PHARMACEUTICAL COMPOSITIONS AND PHARMACEUTICAL FORMS OF THE INVENTION

A **third aspect** of the invention relates to the use of a pharmaceutical composition comprising at least one combined preparation or a compound of the invention, or a tautomer, a pharmaceutically acceptable salt, an analog, or a prodrug thereof, preferably together with a pharmaceutically acceptable carrier or vehicle, and/or one or more excipients, hereinafter pharmaceutical composition of the invention, for use in preventing, alleviating, and/or treating alcohol use disorder.

The pharmaceutically acceptable adjuvants and vehicles that can be used in said compositions are the adjuvants and vehicles known to those skilled in the art and commonly used in the preparation of therapeutic compositions.

In the sense used in this description, the expression "therapeutically effective amount" refers to the amount of the agent or compound capable of developing the therapeutic action determined by its pharmacological properties, calculated to produce the desired effect, and it will generally be determined by, among others, the characteristics typical of the compounds, including patient age and condition, the severity of the disturbance or disorder, and the administration route and frequency.

The compounds described in the present invention, the salts, prodrugs, and/or solvates thereof, as well as the pharmaceutical compositions containing them, can be used together with other additional drugs or active ingredients to provide a combination therapy. Said additional drugs can be part of the same pharmaceutical composition or can alternatively be provided in the form of a separate composition for the simultaneous or non-simultaneous administration thereof with respect to the administration of the pharmaceutical composition comprising a compound of the invention, or a salt, prodrug, or solvate thereof.

Therefore, in a preferred embodiment the pharmaceutical composition further comprises another active ingredient.

As it is used herein, the term "active ingredient," "pharmaceutically active ingredient," "active substance," or "pharmaceutically active substance" means any component which potentially provides a different pharmacological activity or another different effect on the diagnosis, cure, mitigation, treatment, or prevention of a disease, or affects the structure or function of the body of humans or other animals. The term includes those components which promote a chemical change in the preparation of the drug and are present therein in an expected modified form providing the specific activity or effect.

In another preferred embodiment, the pharmaceutical composition of the invention comprises, preferably as the only active ingredients, the PPARα agonist and the CB1 receptor antagonist, or the PPARα agonist and the PPAPγ agonist, or any of the compounds with dual activity described throughout the present specification, although said pharmaceutical composition may additionally comprise pharmaceutically acceptable excipients and/or vehicles. Therefore, the pharmaceutical composition of the invention preferably comprises any of the preparations or compounds of the invention.

Another aspect of the invention relates to a pharmaceutical form, hereinafter pharmaceutical form of the invention, comprising any of the preparations or compounds of the invention.

"Pharmaceutical form" is understood herein to mean the mixture of one or more active ingredients with or without additives presenting physical characteristics for the suitable dosing, storage, administration, and bioavailability thereof.

In another preferred embodiment of the present invention, the compositions and pharmaceutical forms of the invention are suitable for oral administration, in solid or liquid form. The possible forms for oral administration are tablets, capsules, syrups, or solutions and may contain conventional excipients known in the pharmaceutical field, such as binding agents (e.g. syrup, acacia gum, gelatin, sorbitol, tragacanth, or polyvinyl pyrrolidone), fillers (e.g. lactose, sugar, corn starch, calcium phosphate, sorbitol, or glycine), disintegrants (e.g. starch, polyvinyl pyrrolidone, or microcrystalline cellulose), or a pharmaceutically acceptable surfactant such as sodium lauryl sulfate. Other pharmaceutical forms can be colloidal systems, among which polymeric nanoemulsions, nanocapsules, and nanoparticles are included.

The compositions for oral administration can be prepared by conventional galenic pharmacy methods, such as mixture and dispersion. The tablets can be coated following known methods in the pharmaceutical industry.

The compositions and pharmaceutical forms can be adapted for parenteral administration, such as sterile solutions, suspensions, or lyophilisates of the products of the invention, using the suitable dose. Suitable excipients, such as pH buffering agents or surfactants, can be used.

The formulations mentioned above can be prepared using conventional methods, such as those described in the pharmacopoeias of different countries and in other reference texts.

As it is used herein, the term "medicinal product" refers to any substance used for preventing, diagnosing, alleviating, treating, or curing diseases in humans and animals.

The administration of the compounds, compositions, or pharmaceutical forms of the present invention can be performed by means of any suitable method, such as intravenous infusion and oral, topical, or parenteral routes. Oral administration is preferred because of the convenience for patients and the chronic nature of the diseases to be treated.

The administered amount of a compound of the present invention will depend on the relative efficacy of the chosen compound, the severity of the disease to be treated, and the weight of the patient. However, the compounds of this invention will be administered one or more times a day, for example 1, 2, 3, or 4 times a day, with a total dose between 0.1 and 1000 mg/kg/day. It is important to take into account that it may be necessary to introduce variations in the dose, depending on the age and the condition of the patient, as well as modifications in the administration route.

The compounds and compositions of the present invention can be used together with other medicinal products in combined therapies. The other drugs may be part of the same composition or another different composition, for the administration thereof at the same time or at different times.

Another **aspect** of the invention relates to a food composition such as a nutraceutical composition or a medical food- or functional food-type composition, hereinafter food composition of the invention, comprising at least one of the compounds of the invention in an effective amount for preventing, alleviating, and/or treating alcohol use disorder in mammals, including humans.

The preferred food compositions are selected from the list consisting of: a beverage, milk, yogurt, cheese, fermented milk, flavored milk beverage, soy milk, precooked grains, bread, pastries, butter, margarine, sauces, oils used for frying, vegetable oils, corn oil, olive oil, soybean oil, palm oil, sunflower oil, cottonseed oil, condiments, dressings for salads, fruit juices, syrups, desserts, glazes and fillers, soft frozen products, candies, gums, and intermediate foods. The food composition of the invention can be a nutritional or dietary supplement. In another preferred embodiment, the nutritional or dietary supplement comprises a sterile composition containing the compound of the invention, preferably provided with a gastric acid-resistant coating, being a delayed release composition. In another preferred embodiment, the food composition, including the compound of the invention and/or the nutritional or dietary supplement, comprises suitable "carriers" such as diluents, adjuvants, excipients, or vehicles with which the compound of the invention is administered. Suitable excipients include, but are not limited to, starch, glucose, fructose, lactose, sucrose, gelatin, malt, rice, flour, calcium sulfate, silica gel, sodium stearate, glycerol monostearate, talc, sodium chloride, skimmed milk powder, glycerol, propylene, glycol, water, ethanol, and the like. Such nutritional supplements can be used to combat liver problems and help to maintain the health or a healthy lifestyle in mammals, preferably humans.

As it is understood herein, the term "treatment" refers to combating the effects caused as a consequence of a pathological condition or disease of interest in a subject (preferably mammals, and more preferably humans) including:
(i) inhibiting the pathological condition or disease, i.e., stopping its development;
(ii) alleviating the pathological condition or disease, i.e., causing the regression of the pathological condition or disease or its symptomatology;
(iii) stabilizing the pathological condition or disease.

As it is understood herein, the term "prevention" consists of preventing the onset of the disease, i.e., preventing the pathological condition or disease from occurring in a subject (preferably mammals, and more preferably humans), particularly when said subject has a predisposition for the pathological condition.

Throughout the description and claims, the word "comprises" and its variants do not intend to exclude other technical features, additives, components, or steps. For those skilled in the art, other objects, advantages, and features of the invention will be inferred in part from the description and in part from putting the invention into practice. The following examples and figures are provided by way of illustration and are not intended to be limiting of the present invention.

### EXAMPLES OF THE INVENTION

### Material and methods

### Animals

For the alcohol self-administration studies, 48 adult male Wistar rats (8 per group) (Harlan, Barcelona, Spain) weighing 375-425 g at the start of the experiments were individually housed in a 12-hour light/dark cycle (turned off at 12:00 p.m.) in a room at constant temperature (23 ± 1°C). Standard food and tap water were available *ad libitum* in the house cage. The animals were allowed to acclimate to the housing facilities for 2 weeks before the beginning of the alcohol self-administration protocol. For oxycodone self-administration, male Long-Evans rats (275-325 g) (Charles-River Laboratories, Raleigh, N.C.) were used throughout this study. Upon arrival, they were individually housed in a facility for animals under an inverted 12-h light/dark cycle (light turned on at 7:00 p.m.) with free access to food and water. They were allowed to acclimate to the new environment for at least 7 days before the start of the study. For alcohol consumption in the two-bottle choice paradigm, male Wistar rats weighing 250 g at the start of the experiments, or genetically selected Marchigian Sardinian (msP) male rats that prefer alcohol were used.

All the experimental methods using animals were performed in accordance with the European Directive 2010/63/EU on the protection of animals used for scientific purposes and with Spanish regulations (RD 53/2013 and RD 178/2004). All the protocols were approved by the Ethics Committee of Universidad Complutense of Madrid (Alcohol Studies in Wistar rats), or Universidad de Camerino (msP rats). The oxycodone studies were approved by the US National Institute on Drug Abuse (NIDA) and were consistent with the Guide of the Care and Use of Laboratory Animals of the US National Research Council. Special care was taken to minimize the suffering and the number of animals to achieve the present research objectives.

### Drugs

NF 10-360 was synthesized by the Instituto de Química Medica, Consejo Superior de Investigaciones Científicas, Madrid, as described in Fresno et al., 2015 (J Med Chem. 58(16):6639-52). N-[1-(3,4-dihydroxyphenyl)propan-2-yl]oleamide (OLHHA) was synthesized as described in Almeida et al., 2010. ChemMedChem. 5(10): 1781-7. Oxycodone HCI, sucrose, and 2-hydroxypropyl-β-cyclodextrin were acquired from Sigma/RBI (St Louis, MO, USA). Oxycodone was dissolved in physiological saline solution. 2-Hydroxypropyl-β-cyclodextrin was dissolved in 25% 2-hydroxypropyl- β-cyclodextrin in distilled water. The resuscitation drugs (ciglitazone, WY 14643, oleoylethanolamide (OEA), and rimonabant) were acquired from Tocris-Bioscience (Biogen Científica, Madrid, Spain). A fresh solution was prepared daily (before the injection) by dissolving the drugs in the solution of the vehicle (1% Tween 80 in 0.9% saline solution). All the medicinal products were injected in a volume of 2 ml/kg. Doses of OLHHA and NF 10-360 were selected based on prior publications of the research group [24, Fresno et al., 2015. J Med Chem. 27 58(16):6639-52.

### Alcohol self-administration model

Animals were trained for the alcohol self-administration as previously described (Cippitelli et al., 2005. Eur J Neurosci. 21 (8):2243-51), in the room (Letica, model LE 850, Panlab, Barcelona, Spain) enclosed in sound-attenuating cages and equipped with an exhaust fan. The chambers were equipped with two retractable levers, one located on each side of a water pan.

The active lever side is equally distributed between sessions to prevent the development of location preferences. By pressing the 0.1 ml solution active lever, it was presented to the animal followed by a 2.5-second wait time, while pressing the inactive lever gave no results. All the operant alcohol sessions lasted for 30 minutes per day 5 d/week (Monday to Friday). The amount of responses and the immersion presentations were automatically recorded by the computer software. The animals were weighed daily before the alcohol self-administration sessions. Training was carried out using a modification of the conventional saccharin fading method (Samson *et al.,* 1999) described in Alen et al., 2009 (Nicotine Tob Res. 11 (11): 1304-11). For the first 3 days of training, the animals received a 0.2% saccharin solution in the dipper to facilitate the acquisition of the lever pressure level. After that point, the following sequence in a fixed ratio 1 was used: 0.16% saccharin and 2% alcohol for three sessions, 0.12% saccharin and 4% alcohol for three sessions, 0.08% saccharin and 6% alcohol for four sessions, 0.04% saccharin and 8% alcohol for four sessions, 0.02% saccharin plus 10% alcohol, and finally 10% alcohol alone for the remaining sessions. The first investigation was relatively similar, followed by a period of at least 6 weeks with access to alcohol (10% w/v). ciglitazone (0.01, 1.5, and 20 mg/kg), OEA (0.1, 1.5, and 20 mg/kg), WY 14643 (0.1, 5, 20, and 40) mg/kg), rimonabant (0.03, 0.3, 1, and 3 mg/kg), OLHHA (0.01, 0.1, 1, and 5 mg/kg), and NF 10-360 (1 mg/kg). The medicinal products were injected 30 minutes before the self-administration session. The animals were then left for a period of 24 hours, and then 30 minutes daily. The EtOH self-administration sessions were resumed and monitored.

### Alcohol consumption in the two-bottle choice model

Voluntary alcohol intake was evaluated in a two-bottle choice test, as previously described [Stopponi et al., 2011. Biol Psychiatry. 69(7):642-]. Wistar rats or rats selected due to their alcohol preference (msP) were trained to drink water, 10% alcohol (v/v) (Wistar), or 20% alcohol (msP) for 24 hours per day until reaching a stable alcohol intake baseline. At this point, the rats were divided into different groups (n = 9-10/group) to be treated with vehicle, NF 10-360 (0 or 1 mg/kg), or OLHHA (5 mg/kg). In the case of msP rats, treatment with OLHHA continued for 14 consecutive days, and the drug or vehicle was administered once a day before the start of the dark period of the light/dark cycle. Intakes were recorded daily 2, 8, and 24 hours after the administration of OLHHA. Alcohol, water, and food intake were monitored daily. The animals were sacrificed on day 14, 2 hours after the last administration of OLHHA.

### Oxycodone self-administration

Oxycodone self-administration studies were performed as previously described in [You et al., 2017. Neuropharmacology. 126:190-199]. An intravenous (i.v.) catheter (Braintree Scientific, Inc., Braintree, MA, USA) was implanted in the rats used in the oxycodone self-administration experiments. Each rat was first anesthetized with pentobarbital (30 mg/kg i.p.) supplemented with chloral hydrate (140 mg/kg, i.p.), and then a small incision was made to the right of the midline of the neck to expose the external jugular vein. One end of the catheter was on the side of the catheter reaching the right atrium. The catheter is then secured to the vein with a silk suture, and the other end then fed by subcutaneous route into the posterior part of the neck to come out close to the posterior part of the cranium, connected to a bent 24-gauge stainless steel cannula (Plastics One Inc., Roanoke, VA, USA) with a mock cannula and, during experimentation, an infusion line. The catheter and the guide cannula were fixed to the cranium with four stainless steel screws and bolts. The incision was then sutured. After surgery, the catheters were washed daily with a solution of gentamicin-heparin-saline solution (0.1 mg/ml of gentamicin and 30 IU/ml of heparin, ICN Biochemicals, Cleveland, OH, USA) as a precautionary measure against catheter blockage and infection. The animals were allowed to recover for at least 5 days before starting behavioral training.

Oxycodone self-administration is carried out in an operant chamber with two response levers and a 15 W house light (Med Associates Inc., Georgia, VT, USA). The two levers were placed 6.4 cm above the floor of the chamber; one was active while the other one was inactive. They were 12 cm above the active daybreak (Xi and Gardner, 2007). After fully recovering from surgery, the rats were trained to self-administer oxycodone. To that end, they were transported each day of training to the testing room and connected by a polyethylene tube (protected by a steel coil spring) and to a syringe pump with a rotating liquid channel (Razel Sci., Stamford, CT, USA) controlled by a microprocessor, and placed in the chamber. Each training session begins with the presentation of the response to the chamber and the lighting up of the house light, which was maintained at the end of each training session. Each rat has been trained daily in 3-hour sessions for the active person seeking oxycodone infusions in a fixed ratio 1 (FR-1) reinforcement scheme. The response on the active lever resulted in the activation of a signal consisting of a light tone and the infusion of a 0.08 ml oxycodone solution for 4.6 s. This time is also a wait time period during which the light tone is maintained in the response of the animal. The response of each animal has been recorded throughout the entire training and testing process.

### Plasma and liver sampling

The rats were sacrificed 2 hours after the last dose. Treated animals and animals treated with OLHHA were anesthetized with sodium pentobarbital (50 mg kg-1, i.p.), and blood and liver samples were taken. The blood was centrifuged (2100 g for 8 min, 4°C) and the plasma was kept for later analysis. The liver samples were rapidly frozen in liquid N²and stored at -80°C until analysis.

### Plasma metabolite and cytokine measurements

The following metabolites, metabolic hormones, and cytokines were measured in plasma: metabolites glucose, triglycerides, cholesterol, uric acid, urea, creatinine, aspartate transaminase (GOT), alanine transaminase (GPT), interleukin-6, tumor necrosis factor α (TNF-a). They were analyzed in an automatic Hitachi 737 analyzer (Hitachi, Tokyo, Japan) according to the manufacturer's instructions. Levels of leptin, IL-6, and TNF-α were measured using commercial rat enzyme-linked immunosorbent assay kits (Abcam, Cambridge, United Kingdom).

### Isolation of RNA and RT qPCR analysis

Real-time quantitative polymerase chain reaction (RT qPCR) was used to measure the relative quantification of the mRNA levels of the enzymes and proteins related to the oxidation of hand lipogenic fatty acids, as described in previous studies with OLHHA. [Decara et al., 2015. Dis Model Mech. 8(10): 1213-25]. Total mRNA was isolated using the Trizol® method according to the manufacturer's instructions (Gibco BRL Life Technologies, Baltimore, MD, USA). Resulting cDNA was used as templates for RT qPCR with an iCycler system (BioRad) using the Quanti-Test SYBR Green PCR kit (Qiagen, Hilden, Germany). The authors were provided by Sigma-Proligo (Proligo France SAS, Paris, France). Quantification was performed according to the following criteria [Decara et al., 2015. Dis Model Mech. 8(10): 1213-25]. The absolute values of each sample were normalized to observe β-actin mRNA (constitutive gene), which was used as a standard reference.

### Data analysis

GraphPad Prism v5.04 (GraphPad Software, San Diego, CA, USA). Data was represented by means ± SEM. EC50 values for the inhibition of alcohol, cocaine, or oxycodone self-administration were calculated using built-in non-linear regression equations. Supplementation with alcohol and the analysis of the effects of variability (ANOVA) with repeated measures (factors: treatment and treatment). Plasma metabolic parameters, cytokines, and liver mRNA resulting from the circulation expression were analyzed by means of a two-way ANOVA (factors: treatment (vehicle/OLHHA) and access to beverage (octanol/water)), and a multiple *post hoc* comparison test (Bonferroni) was performed. The p-value less than 0.05 was considered to be statistically significant.

### Results

### 1. Actions of the reference ligands for the PPARα, PPARy, and CB1 receptors in alcohol self-administration (Figure 1)

For a comparative overview of the efficacy of the reference agonists for the PPARα, PPARy, and CB1 receptors and antagonists for the cannabinoid receptors in alcohol self-administration, the EC50 and maximum ciglitazone inhibition (PPARγ agonist reference), OEA (endogenous ligand for PPARα receptors), WY14643 (reference standard for PPARα ligand receptors), and rimonabant (SR141716A, the reference cannabinoid receptor antagonist/inverse agonist, also coded) were calculated. Of these compounds, rimonabant achieved maximum inhibition, followed by OEA, ciglitazone, and WY14643. PPARα and PPARα receptor agonists, which are selectively of interest, seem to be limited in terms of the inhibitory effect of alcohol self-administration, achieving maximum dose effects at 20 mg/kg. EC50 values for the effects of those compounds (IC50): ciglitazone (Figure 1A): EC50 = 5.9 (IC = 2.7 to 15.7); OEA (Figure 1B): EC50 = 6.5 (IC = 3.2 to 13.2); WY14643 (Figure 1C): EC50 = 23.6 (IC = 11.3 to 60.9); Rimonabant (Figure 1D): EC50 = 0.5 (IC = 0.5 to 1.1).

### 2 Effects of the double PPARα/γ agonist NF 10-360 on alcohol intake and alcohol self-administration (Figure 2)

A dose of 1 mg/kg of the compound NF 10-360 is used to analyze the effects of this compound on voluntary alcohol intake (Figure 2A) and self-administration (Figure 2B). The data indicates that this compound reduced alcohol intake, F (1.84) = 4.33, P <0.05. This compound also reduced alcohol self-administration studies, wherein NF 10-360 reduced operant responses to a 10% ethanol solution, t = 3.26, df = 10, number of pairs = 11, P <0.01.

### 3 Effects of the dual ligand of the PPARα/CB₁ receptor OLHHA on alcohol self-administration (Figures 3A and 3B)

Effects of PPARα agonist CB1 receptor antagonist OLHHA on ethanol self-administration (Figures 3A and 3B) in adult male Wistar rats were studied. This is a reduced dose-dependent response rate for a maximum dose of 5 mg/kg (Figure 3B). EC50 = 0.2 mg/kg (IC = 0.08 to 0.4). This effect did not affect alcohol self-administration when analyzed 24 hours after administration (Figure 3B).

### 4 Effects of the dual ligand of the PPARα/CB1 receptor OLHHA on voluntary alcohol intake in msP rats that prefer alcohol (Figures 3C to 3F)

In this study, the effects of OLHHA on the two-bottle choice paradigm are reported and the effects of the daily administration of 5 mg/kg 2, 8, and 24 hours after administration are studied (Figures 3C to 3F). OLHHA reduced alcohol intake 2 hours after injection, F (1.14) = 39.5, P <0.0001, (Figure 3C). This effect was also observed 8 h after the administration of OLHHA, F (1.14) = 23.9, P <0.001, (Figure 3D), and virtually disappeared after 24 h, F (1.14) = 4.7, P = 0.047, (Figure 3E), suggesting that the pharmacological effects of OLHHA have a time limit. This observation is according to the absence of effects of OLHHA on alcohol self-administration when this behavior is tested 24 hours after administration (Figure 4). Total alcohol intake recorded throughout treatment with OLHHA was reduced significantly, in any case, F (1.14) = 5.1, P <0.05 (Figure 3F), indicating a long-term reduction in alcohol intake as the result of daily treatment with OLHHA when ethanol is made available continuously.

### 5 Effects of the dual ligand of the PPARα/CB1 receptor OLHHA on oxycodone self-administration (Figure 3G)

Due to the known inhibitory actions of the administration of the cannabinoid CB1 receptor on opioid self-administration, it was tested whether OLHHA was capable of reducing oxycodone self-administration in Long-Evans rats. OLHHA reduced oxycodone self-administration with a maximum inhibition reaching 45% of the control responses for this opioid. The calculated EC50 was 11.3 mg/kg (IC = 5.1 to 25.1). This data suggests that the OLHHA potency in reducing oxidation is at least 50% lower than that observed for alcohol. It should be pointed out that it has been proven that PPARα agonism affects the actions of opioids in the dopaminergic circuits of CB1 receptor antagonism.

### 6 Effects of the repeated administration of the dual ligand of the PPARα/CB1 receptor OLHHA on plasma and liver metabolic parameters in msP rats that drink alcohol (Figure 4)

Since the administration of alcohol may have a harmful impact on liver metabolism and it was reported that OLHHA produces and improvement in lipid metabolism in the liver of fatty rats with leptin deficiency, it was tested whether the administration of OLHHA may have an impact on liver metabolism, eventually improving or impairing ethanol toxicity (see Figure 4). The results obtained indicate that, as previously described in fatty rats with leptin deficiency, treatment with OLHHA reduced circulating triglycerides, F (1.28) = 25.6, P <0.001, (Figure 4B), cholesterol, F (1.28) = 25.4, P <0.001, (Figure 4C), and uric acid, F (1.28) = 17.4, P <0.01, (Figure 4D), slightly reducing glucose levels (Figure 4A) in rats exposed to alcohol F (1.28) = 5.6, P <0.02. OLHHA did not induce toxicity (no impact on transaminases (Figures 4G and 4H) or on creatinine (Figure 4F)) or inflammation (no effect on both plasma concentrations of IL-6 (Figure 4I) and on plasma concentrations of TNF (Figure J)). Furthermore, as described for most PPAR agonists and CB1 antagonists, OLHHA inhibited the expression of mRNA of lipogenic enzymes such as Fasn, F (1.28) = 25.5, P <0.001 (Figure 4M) and SCD1, F (1.28) =13.1, P <0.01 (Figure L), and increased the expression of cytochrome c oxidase 4, F (1.28) =10.3, P <0.01 (Figure 4N) and PPAR receptor, F (1.28) = 23.6, P <0.001, (Figure 4O), suggesting the promotion of oxidative metabolism.

## Claims

1. A composition comprising at least one PPARα agonist compound, wherein said composition is **characterized in that** it comprises a second compound with CB1 receptor antagonist activity or PPARψ agonist activity, or wherein said composition is **characterized in that** said compound exhibits, simultaneous to PPARα agonist activity, CB1 receptor antagonist activity or PPARψ agonist activity,
for use in preventing, alleviating, improving, and/or treating alcohol use disorder.

2. The composition for use according to claim 1, wherein said composition is a combined preparation comprising at least one PPARα agonist compound and a compound with CB1 receptor antagonist activity.

3. The composition for use according to claim 1, wherein said composition is a combined preparation comprising at least one PPARα agonist compound and a compound with PPARψ agonist activity.

4. The composition for use according to claim 1, wherein said composition is **characterized in that** said compound exhibits, simultaneous to PPARα agonist activity, CB1 receptor antagonist activity.

5. The composition for use according to claim 1, wherein said composition is **characterized in that** said compound exhibits, simultaneous to PPARα agonist activity, PPARψ agonist activity.

6. The composition for use according to any of claims 2 or 4, wherein:
a. the PPARα agonist is selected from the list consisting of clofibrate, gemfibrozil, fenofibrate, elafibranor, prasterone, ciprofibrate, oleoylethanolamide, CP 775146, GW 7647, WY 14643, or any pharmaceutically acceptable salts thereof, and wherein
b. the CB1 receptor antagonist is selected from antagonists AVE-1625, CP-945598, SLV319, V24343 AM 251, AM 4113, AM 6545, CP 945598 hydrochloride, MJ 15, NIDA 41020, PF 514273, (±)-SLV 319, propacetamol, tetrahydrocannabivarin, or inverse agonists selected from AM 281, hemopressin, LY 320135, SR 141716A, TC-C 14G, or any pharmaceutically acceptable salts thereof.

7. The composition for use according to any of claims 3 or 5, wherein:
a. the PPARα agonist is selected from the list consisting of clofibrate, gemfibrozil, fenofibrate, elafibranor, prasterone, ciprofibrate, oleoylethanolamide, CP 775146, GW 7647, WY 14643, or any pharmaceutically acceptable salts thereof, and wherein
b. the PPAPγ agonist is selected from the list consisting of: (2S)-2-(4-chlorophenoxy)-3-phenylpropanoic acid, (2S)-2-(biphenyl-4-yloxy)-3-phenylpropanoic acid, (2S)-2-ethoxy-3-{4-[2-(10H-phenoxazin-10-yl)ethoxy]phenyl}propanoic acid, (S)-3-(4-(2-carbazol-9-yl-ethoxy)-phenyl)-2-ethoxy-propionic acid, 2-chloro-5-nitro-N-phenylbenzamide, 2-{5-[3-(7-propyl-3-trifluoromethylbenzo[D]isoxazol-6-yloxy)propoxy]indol-1-yl}ethanoic acid, 3-(5-methoxy-1 H-indol-3-yl)propanoic acid, 3-[5-(2-nitropent-1-en-1-yl)furan-2-yl]benzoic acid, 3-fluoro-N-[1-(4-fluorophenyl)-3-(2-thienyl)-1 H-pyrazol-5-yl]benzenesulfonamide, balsalazide, glipizide, mesalazine, mitiglinide, nateglinide, repaglinide, sulfasalazine, T131, telmisartan, baroxolone, thiazolidinone, 15-deoxy-delta 12,14-prostaglandin J2, S26948, nTZDpa, LG 100754, GW 1929 hydrochloride, edaglitazone, ciglitazone, inolitazone, SR 2595, GW1929, or any pharmaceutically acceptable salts thereof.

8. The compound simultaneously exhibiting PPARα agonist activity and CB1 receptor antagonist activity for use according to claim 4, wherein said compound is N-[1-(3,4-dihydroxyphenyl)propan-2-yl]oleamide (OLHHA), or any pharmaceutically acceptable salts thereof.

9. The compound simultaneously exhibiting PPARα agonist activity and PPAPγ agonist activity for use according to claim 5, wherein said compound is the compound 3-((4-benzyl-2-oxooxazolidin-3-yl)methyl)-N-(4-(pentylcarbamoyl)phenyl)benzamide (NF 10-360), or any pharmaceutically acceptable salts thereof.

10. The composition according to any of claims 1 to 9, wherein said composition is a pharmaceutical composition for use in preventing, alleviating, and/or treating alcohol use disorder.

11. The composition according to any of claims 1 to 9, wherein said composition is a food composition for use in preventing, alleviating, and/or treating alcohol use disorder.
